(19) Europäisches Patentamt
European Patent Office
Office européen des brevets

(11) Veröffentlichungsnummer: **0 150 411**
**A1**

(12) # EUROPÄISCHE PATENTANMELDUNG

(21) Anmeldenummer: **84115610.2**

(22) Anmeldetag: **17.12.84**

(51) Int. Cl.⁴: **C 07 D 239/96**

(30) Priorität: **30.12.83 DE 3347526**

(43) Veröffentlichungstag der Anmeldung: **07.08.85 Patentblatt 85/32**

(84) Benannte Vertragsstaaten: **AT BE CH DE FR GB IT LI NL SE**

(71) Anmelder: **Troponwerke GmbH & Co. KG, Berliner Strasse 156, D-5000 Köln 80 (DE)**

(72) Erfinder: **Opitz, Wolfgang, Dr., Holzbachtalstrasse 60, D-5063 Overath (DE)**

(74) Vertreter: **Adrian, Albert, Dr. et al, c/o BAYER AG Konzernverwaltung RP Patentabteilung, D-5090 Leverkusen 1, Bayerwerk (DE)**

(54) **Verfahren zur Herstellung substituierter Chinazolin-2.4(1H.3H)-dione.**

(57) Die vorliegende Erfindung betrifft ein neues Verfahren zur Herstellung substituierter Chinazolin-2.4(1H.3H)-dione der allgemeinen Formel I

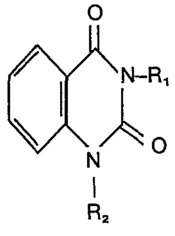

I

in der

$R_1$ für Wasserstoff, niedriges Alkyl, ungesättigtes niedriges Alkenyl, substituiertes niedriges Alkyl oder Aralkyl und

$R_2$ für einen gegebenenfalls substituierten Arylrest steht

unter Bedingungen der Phasentransferkatalyse.

EP 0 150 411 A1

 0150411

TROPONWERKE GmbH & Co. KG

5000 Köln 80
Je/by-c

# Verfahren zur Herstellung substituierter Chinazolin-2.4(1H.3H)-dione

Die vorliegende Erfindung betrifft ein neues Verfahren zur Herstellung substituierter Chinazolin-2.4(1H.3H)-dione der allgemeinen Formel I

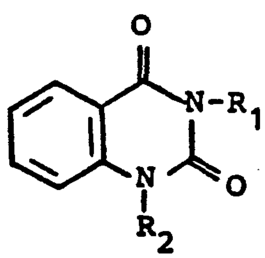

I

in der

$R_1$ für Wasserstoff, niedriges Alkyl, ungesättigtes niedriges Alkyl, substituiertes niedriges Alkyl oder Aralkyl und

$R_2$ für einen gegebenenfalls substituierten Arylrest steht

unter Bedingungen der Phasentransferkatalyse.

TP 71-Ausland

Bevorzugt wird das erfindungsgemäße Verfahren ausgeübt, um Verbindungen der allgemeinen Formel I zu erhalten, worin

$R_1$ die Bedeutung von Wasserstoff, niedrigen Alkylgruppen mit 1 bis 6 Kohlenstoffatomen, niedrigen Alkenylgruppen mit 3 bis 5 Kohlenstoffatomen, Propargyl, Cyclopropyl, Cyclopropylmethyl, Halogenethyl, Trihalogenethyl, Acetoxyethyl, Alkoxyalkylgruppen mit 2 bis 4 Kohlenstoffatomen, niedrigen Hydroxyalkylgruppen mit 2 bis 3 Kohlenstoffatomen, Hydroxyethoxyethyl, Ethoxycarbonylmethyl oder Benzyl hat und

$R_2$ für einen Phenylrest steht, der durch Nitro- oder halogenierte - bevorzugt fluorierte - niedrige Alkylgruppen, bevorzugt die $CF_3$-Gruppe, Alkylthio- oder Alkoxygruppen mit bis zu drei C-Atomen substituiert sein kann.

Besonders bevorzugt wird nach dem erfindungsgemäßen Verfahren 1-(3-Nitrophenyl)-3-ethylchinazolin-2.4(1H.3H)-dion hergestellt.

Für die Herstellung von Verbindungen der allgemeinen Formel I sind bereits Verfahren beschrieben worden. Ein Verfahren besteht in der Umsetzung substituierter Anthranilsäureamide mit geeigneten Kohlensäurederivaten (vgl. DE-OS 2 459 090). Dabei läßt man die entsprechenden Anthranilsäureamide zunächst in einem

absoluten Lösungsmittel mit Alkalimetallen oder Natriumamid, -hydrid oder -alkoholat reagieren. Dies ist bei großtechnischen Verfahren problematisch. Anschließend wird mit geeigneten Kohlensäurederivaten umgesetzt. Diese müssen in der Regel im mehrfach molaren Überschuß eingesetzt werden, um gute Ausbeuten zu erzielen. Dies bekannte Verfahren arbeitet also nicht wirtschaftlich. Ferner müssen die Überschüsse aufgearbeitet oder umweltneutral vernichtet werden. Dies stellt eine weitere Belastung des Verfahrens dar.

Überraschenderweise wurde nun gefunden, daß man Verbindungen der allgemeinen Formel I durch

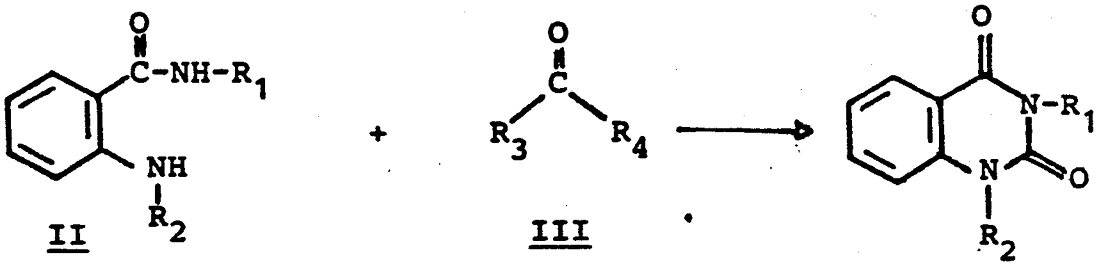

II + III → I

Umsetzung von Verbindungen der allgemeinen Formel II, in denen

$R_1$ und $R_2$ die bei der allgemeinen Formel I angegebenen Bedeutungen haben,

mit lediglich molaren Mengen bzw. einem geringen Überschuß an Verbindungen der allgemeinen Formel III, in der

$R_3$ Halogen, vorzugsweise Chlor oder Brom und

$R_4$ Halogen, Alkoxy oder Aryloxy, vorzugsweise Chlor oder Ethoxy bedeutet,

in Gegenwart von wäßriger Base und einem geeigneten, mit Wasser praktisch nicht mischbaren organischen Lösungsmittel unter milden Bedingungen und in hoher Ausbeute und Reinheit herstellen kann, wenn man unter den Bedingungen der Phasentransferkatalyse arbeitet (vgl. u. a. Angew. Chem. 89, 521 (1977)).

Erfindungsgemäß wird unter einem mit Wasser praktisch nicht mischbaren Lösungsmittel ein unpolares, aprotisches Lösungsmittel, wie z. B. halogenierte oder aromatische Kohlenwasserstoffe, verstanden. Als erfindungsgemäß besonders geeignet haben sich hierbei Dichlormethan und Toluol erwiesen. In diesem Lösungsmittel werden Anthranilsäurederivate der allgemeinen Formel II gelöst. Nach Zusatz eines Phasentransferkatalysators wird mit einer geeigneten Base versetzt und das zweiphasige Gemisch einige Minuten stark gerührt. Als Phasentransferkatalysatoren eignen sich Tetraalkylammonium-, Tetralkylphosphonium-, Tetraalkylarsonium- oder Trialkylsulfoniumsalze, Kronenether, Kryptate, Polyethylenglykole und deren Ether; als erfindungsgemäß besonders günstig haben sich Tetraalkylammoniumsalze, besonders Tetrabutylammoniumhydrogensulfat, erwiesen. Bevorzugte Basen sind Alkalihydroxide, als besonders geeignet hat sich ca. 50 gew.-%ige wäßrige Natronlauge erwiesen.

Anschließend wird das Reaktionsgemisch mit Verbindungen der allgemeinen Formel III, in der

$R_3$ und $R_4$ die obengenannte Bedeutung haben - bevorzugt sind Chlorameisensäureester, besonders der Ethylester oder Phosgen, die in einem geeigneten Lösungsmittel gelöst sind -

versetzt. Dabei kann das zum Lösen von III benutzte Lösungsmittel gleich oder verschieden von dem sein, das zum Lösen von II verwendet wird, es muß jedoch auch möglichst unpolar, aprotisch und praktisch nicht mit Wasser mischbar sein. Als besonders geeignet haben sich auch hier Dichlormethan und Toluol erwiesen. Die Zugabe von III kann auf einmal oder in mehreren Portionen vorgenommen werden, in einzelnen Fällen kann die Zugabe von mehr als einem Mol III pro Mol II erforderlich sein; bevorzugt ist ein Überschuß von 5 % - 20 %. Die Reaktion wird unter starkem Rühren mit einem Magnetrührer über einen Zeitraum von 10 Min. bis 2 Stunden, bevorzugt über 30 - 40 Min. bei Temperaturen zwischen ca. 10 - ca. 35°C, bevorzugt bei Raumtemperatur durchgeführt. Unter Raumtemperatur werden Temperaturen zwischen ca. 18°C und 25°C verstanden. Nach beendeter Reaktion wird das Reaktionsgemisch mit Wasser und dem verwendeten organischen Lösungsmittel verdünnt, die organische Phase abgetrennt, mit Wasser gewaschen, getrocknet, eingedampft und der Rückstand in geeigneter Weise vorzugsweise durch Kristallisation oder Chromatographie, gereinigt.

Die vorliegende Erfindung soll durch die folgenden Beispiele näher erläutert werden:

Beispiel 1:

Eine Lösung von 712,5 mg (2,5 mMol) 2-(3-Nitrophenylamino)benzoesäureethylamid in 7,5 ml Dichlormethan wurde bei Raumtemperatur mit 42,4 mg (0,125 mMol) Tetrabutylammoniumhydrogensulfat und 2,5 ml 50 %iger wäßriger NaOH versetzt und das Gemisch 5 Minuten stark gerührt. Anschließend wurden innerhalb 10 Minuten unter starkem Rühren 2,625 ml einer 1 m Lösung von Chlorameisensäureethylester in Dichlormethan zum Reaktionsgemisch getropft und das Rühren 30 Minuten fortgesetzt. Man verdünnte mit je 15 ml Wasser und Dichlormethan, trennte die organische Phase ab, extrahierte die wäßrige Phase mit weiteren 5 ml Dichlormethan und wusch die vereinigten organischen Phasen mit 15 ml Wasser. Zur Entfernung polarer Verunreinigungen wurde die organische Phase mit 4 g Kieselgel 60 (Merck) behandelt, filtriert, der Kieselgelniederschlag mit 80 ml Dichlormethan ausgewaschen und die vereinigten Filtrate i. Vak. eingedampft. Durch Verrühren des Eindampfrückstandes mit Ether wurden 641 mg (82,5 % d. Th.) 1-(3-Nitrophenyl)-3-ethylchinazolin-2.4(1H.3H)-dion vom Fp. 187 - 88 °C erhalten.

Beispiel 2:

Bei Verwendung von Cetyltrimethylammoniumbromid anstelle von Tetrabutylammoniumhydrogensulfat wurden bei ansonsten gleicher Reaktionsführung 50 % d. Th. Produkt vom Fp. 187 - 88 °C erhalten.

Beispiel 3:

Bei Verwendung von Benzyltributylammoniumbromid anstelle von Tetrabutylammoniumhydrogensulfat wurden bei ansonsten gleicher Reaktionsführung 72 % d. Th. Produkt vom Fp. 187 - 88 °C erhalten.

Beispiel 4:

Eine Lösung von 2.85 g (10 mMol) 2-(3-Nitrophenylamino)-benzoesäureethylamid in 30 ml Dichlormethan wurde bei Raumtemperatur mit 169 mg (0.5 mMol) Tetrabutylammoniumhydrogensulfat und 10 ml 50 %iger wäßriger Natronlauge versetzt und 30 Min. stark gerührt. Nach Zusatz von 7.5 ml einer Lösung, die 146 g Phosgen je Liter Toluol enthielt (11 mMol), wurde 15 Min. gerührt und wie unter Beispiel 1 angegeben aufgearbeitet.

Ausbeute: 2.4 g (77.2 % d. Th.) 1-(3-Nitrophenyl)-3-ethylchinazolin-2.4(1H.3H)-dion vom Fp. 185 - 88 °C.

Patentansprüche

1. Verfahren zur Herstellung substituierter Chinazolin-2.4(1H.3H)-dione der allgemeinen Formel I

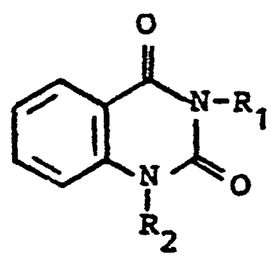

(I)

in welcher

$R_1$ für Wasserstoff, niedriges Alkyl, ungesättigtes niedriges Alkyl, substituiertes niedriges Alkyl oder Aralkyl und

$R_2$ für einen gegebenenfalls substituierten Arylrest steht,

unter Bedingungen der Phasentransferkatalyse.

2. Verfahren nach Anspruch 1, dadurch gekennzeichnet, daß $R_1$ für Wasserstoff, Alkyl mit 1 bis 6 C-Atomen, vorzugsweise mit 1 bis 4 C-Atomen; ungesättigtes Alkyl mit 3 bis 5 C-Atomen, vorzugsweise Propargyl; Cyclopropyl, Cyclopropylmethyl, Halogenethyl, Trihalogenethyl, Acetoxyethyl; Alkoxyalkylgruppen mit 2 bis 4 C-Atomen; Hydroxyalkylgruppen mit 2 bis 3 C-Atomen, vorzugsweise Hydroxyethoxyethyl; Ethoxycarbonylmethyl oder Benzyl und

$R_2$ für einen Phenylrest steht, der durch Nitro-, halogenierte - bevorzugt fluorierte - niedrige Alkylgruppen, vorzugsweise mit 1 bis 4 C-Atomen, besonders bevorzugt durch die Trifluormethylgruppe, Alkylthio- oder Alkoxygruppen mit jeweils bis zu 3 C-Atomen substituiert sein kann.

3. Verfahren zur Herstellung von 1-(3-Nitrophenyl)-3-ethylchinazolin-2.4(1H.3H)-dion der Formel

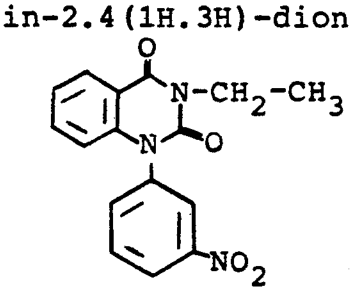

unter Bedingungen der Phasentransferkatalyse.

4. Verfahren zur Herstellung substituierter Chinazolin-2.4(1H.3H)-dione gemäß einem oder mehreren der Ansprüche 1 bis 3, dadurch gekennzeichnet, daß man Verbindungen der allgemeinen Formel II

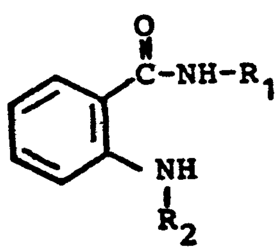

II

in welcher

$R_1$ für Wasserstoff, niedriges Alkyl, ungesättigtes niedriges Alkyl, substituiertes niedriges Alkyl oder Aralkyl und

$R_2$ für einen gegebenenfalls substituierten Arylrest steht,

mit etwa molaren Mengen von Verbindungen der allgemeinen Formel III

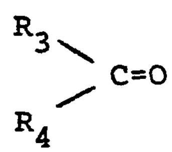

III

in welcher

$R_3$ Halogen, vorzugsweise Chlor oder Brom und

$R_4$ Halogen, vorzugsweise Chlor oder Brom oder Alkoxy oder Aryloxy bedeutet,

in Gegenwart einer wäßrigen Base und einem mit Wasser praktisch nicht mischbaren organischen Lösungsmittel, in Gegenwart eines Phasentransferkatalysators unter heftigem Rühren umsetzt.

5. Verfahren zur Herstellung substituierter Chinazolin-2.4(1H.3H)-dione gemäß einem oder mehreren der Ansprüche 1 bis 4, dadurch gekennzeichnet, daß man die Umsetzung in einem unpolaren, aprotischen Lösungsmittel, und unter Zuhilfenahme eines Phasentransferkatalysators und Alkalihydroxid vornimmt.

6. Verfahren nach einem oder mehreren der Ansprüche 4 bis 5, dadurch gekennzeichnet, daß man als mit Wasser praktisch nicht mischbare Lösungsmittel eines oder mehrere aus der Gruppe halogenierte oder aromatische Kohlenwasserstoffe, vorzugsweise Dichlormethan und/oder Toluol; als Phasentransferkatalysator einen oder mehrere aus der Gruppe Tetraalkylammonium-, Tetraalkylphosphonium-, Tetraalkylarsonium, Trialkylsulfoniumsalze, Kronenether, Kryptate, Polyethylenglykole und deren Ether, bevorzugt Tetraalkylammoniumsalzen, besonders bevorzugt Tetrabutylammoniumhydrogensulfat, einsetzt.

7. Verfahren nach einem oder mehreren der Ansprüche 4 bis 6, dadurch gekennzeichnet, daß man die Verbindungen der Formel III in bis zu einem Überschuß von 25 Mol-%, vorzugsweise 5 bis 20 Mol-% bezogen auf eingesetzte Verbindung II zugibt.

8. Verfahren nach einem oder mehreren der Ansprüche 4 bis 7, dadurch gekennzeichnet, daß man die Reaktion unter heftigem Rühren bei Temperaturen zwischen ca. 10°C bis ca. 35°C, bevorzugt bei Raumtemperatur durchführt.

9. Verfahren nach einem oder mehreren der Ansprüche 4 bis 8, dadurch gekennzeichnet, daß man als wäßrige Base ca. 50 gew.-%ige wäßrige Natronlauge einsetzt.

10. Verfahren nach einem oder mehreren der Ansprüche 4 bis 9, dadurch gekennzeichnet, daß man die Umsetzung von Verbindungen der allgemeinen Formel II mit denen der allgemeinen Formel III unter heftigem Durchmischen bei Temperaturen zwischen 10 bis 35°C, einem Überschuß der Verbindung III von 5 bis 20 Mol-% in Gegenwart von Dichlormethan und/oder Toluol; Tetraalkylammoniumsalzen, bevorzugt Tetrabutylammoniumhydrogensulfat und einer ca. 50 %igen wäßrigen Natronlauge durchführt.

Europäisches Patentamt

# EUROPÄISCHER RECHERCHENBERICHT

0150411 Nummer der Anmeldung

EP 84 11 5610

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| | | | C 07 D 239/96 |
| D,A | DE-A-2 459 090 (HISAMITSU PHARMACEUTICAL CO., INC.) * Ansprüche; Seite 5, Reaktionsschema (III); Seite 6, erster Absatz; Tabelle I, Verbindung Nr. 3 * | 1-4 | |
| A | US-A-3 828 042 (G.F. SCHLAUDECKER et al.) * Ansprüche 1,2 * | 1 | |
| A | DE-A-2 652 144 (MERCK PATENT GMBH) * Seite 10, Zeilen 4,5; Seite 12, Zeilen 7-11 * | 1 | |
| D,A | ANGEWANDTE CHEMIE, Band 89, Nr. 8, 1977; E.V. DEHMLOW "Fortschritte der Phasentransfer-Katalyse", Seiten 521-533 | | RECHERCHIERTE SACHGEBIETE (Int. Cl.4) |
| | | | C 07 D 239/00 |

Der vorliegende Recherchenbericht wurde für alle Patentansprüche erstellt.

| Recherchenort | Abschlußdatum der Recherche | Prüfer |
|---|---|---|
| BERLIN | 22-03-1985 | HASS C V F |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : *in der Anmeldung angeführtes Dokument*
L : aus andern Gründen angeführtes Dokument

& : Mitglied der gleichen Patentfamilie, übereinstimmendes Dokument